Europäisches Patentamt

European Patent Office

Office européen des brevets

(19)

(11) EP 0 563 447 B1

(12) ## EUROPÄISCHE PATENTSCHRIFT

(45) Veröffentlichungstag und Bekanntmachung des Hinweises auf die Patenterteilung:
**22.05.1996 Patentblatt 1996/21**

(51) Int Cl.$^6$: **G01N 33/20**, G01N 33/00

(21) Anmeldenummer: **92120371.7**

(22) Anmeldetag: **28.11.1992**

(54) **Verfahren zur Bestimmung der Konzentration eines Gases in einer Metallschmelze**

Method for determining the concentration of a gas in a molten metal

Procédé de détermination de la concentration d'un gaz dans un métal liquide

(84) Benannte Vertragsstaaten:
**AT BE DE ES FR GB IT LU NL SE**

(30) Priorität: **03.04.1992 DE 4211041**

(43) Veröffentlichungstag der Anmeldung:
**06.10.1993 Patentblatt 1993/40**

(73) Patentinhaber: **Heraeus Electro-Nite International N.V.**
**B-2000 Antwerpen (BE)**

(72) Erfinder: **Plessers, Jaques Josef**
**B-3530 Houthalen (BE)**

(74) Vertreter: **Kühn, Hans-Christian**
**Heraeus Holding GmbH,**
**Stabsstelle Schutzrechte,**
**Heraeusstrasse 12-14**
**63450 Hanau (DE)**

(56) Entgegenhaltungen:
**EP-A- 0 238 054        WO-A-88/07197**
**DE-A- 1 939 403        DE-C- 4 012 454**

## Beschreibung

Die Erfindung betrifft ein Verfahren zu Bestimmung der Konzentration eines Gases in einer Metallschmelze mittels eines Trägergases, das mindestens einem Analysator wahlweise entweder zur Kalibrierung auf direktem Weg oder zur Messung über einen Meßkreis mit einer in die Metallschmelze eingetauchten Meßsonde zugeführt werden kann, wobei das Trägergas zur Messung mit der Metallschmelze in einen Gasaustausch tritt und dieses Gasgemisch dem Analysator zugeführt wird.

Ein derartiges Verfahren ist aus der EP 0 238 054 bekannt. In dieser Veröffentlichung ist die Messung der Konzentration von Wasserstoff, Stickstoff oder Sauerstoff in Stahlschmelzen beschrieben. Hier wird aus einer Gasquelle, die ein Trägergas, beispielsweise Helium oder ein Gemisch aus Helium und dem zu messenden Gas enthält, in einer ersten Phase Trägergas über ein Mehrwegventil durch einen Analysator geleitet, wobei der Analysator gespült und kalibriert wird. Anschließend wird das Trägergas aus der Gasquelle durch eine in der Metallschmelze befindliche Sonde geleitet, in der sie in Gasaustausch mit dem aus der Metallschmelze in die Sonde eindiffundierenden, zu messenden Gas tritt. Anschließend wird dieses Gasgemisch über das Mehrwegventil dem Analysator zugeführt. Das hier beschriebene Meßverfahren hat den Nachteil, daß ein Gasaustausch mit der Metallschmelze erst nach Kalibrierung des Analysators stattfindet. Das zu messende Gasgemisch steht also nicht sofort nach Kalibrierung zur Verfügung, da der Weg des Gases durch den die Meßsonde enthaltenden Meßkreis mehrere Sekunden benötigt, wobei die erste, den Meßkreis durchströmende Gasmenge zur Messung nicht geeignet ist, da der Gasaustausch in dem Meßkreis ebenfalls eine längere Zeit benötigt, so daß die anfangs den Analysator durchströmende Gasmenge nicht den Gehalt des zu messenden Gases repräsentiert. Bei diesem Meßverfahren muß die Meßsonde relativ lange in der Metallschmelze verweilen. Um nicht vorzeitig auszufallen, muß hierbei die Sonde sehr widerstandsfähig gegen die Metallschmelze gestaltet werden, d. h. in der Regel, daß entweder sehr teure Materialien oder kostengünstigere Materialien in einer größeren Stärke verwendet werden müssen. Bei der dadurch entstehenden größeren Masse der Meßsonde ist dann jedoch auch die Temperaturbeeinflussung der Metallschmelze höher, so daß sich die Gleichgewichtsverhältnisse ändern und die Messung ungenau werden kann.

Aufgabe der vorliegenden Erfindung ist es daher, ein Verfahren bereitzustellen, mit dem es möglich ist, die Konzentration eines Gases in einer Metallschmelze mit einer hohen Geschwindigkeit und sehr genau zu bestimmen.

Die Aufgabe wird für ein Verfahren gemäß dem Oberbegriff des Anspruches 1 erfindungsgemäß dadurch gelöst, daß für einen Meßzyklus in einem Meßabschnitt das Trägergas gleichzeitig sowohl direkt dem Analysator zur Kalibrierung als auch dem Meßkreis zur Spülung und zur Aufnahme des zu messenden Gases zugeführt wird derart, daß das zu messende Gas am Analysator bereit steht, daß anschließend das Trägergas über den Meßkreis mindestens teilweise dem mindestens einen Analysator zugeführt wird und daß ein Partialdruck als erster Näherungswert für den Partialdruck des zu messenden Gases in der Metallschmelze nach dem Gasaustausch der Metallschmelze berechnet wird. Durch einen solchen Verfahrensablauf wird der Analysator kalibriert, während gleichzeitig der Meßkreis gespült und das zu messende Gas aus der Metallschmelze aufgenommen wird, so daß nach Beendigung des Kalibrierens sofort und ohne unnötige Zeitverzögerung das zu messende Gas in dem Analysator gemessen werden kann. Es besteht auch die Möglichkeit, mehrere Analysatoren zu verwenden, nämlich beispielsweise einen für das dem Analysator direkt zugeleitete Gas und einen zweiten für das den Meßkreis durchlaufende und das zu messende Gas enthaltende Gasgemisch. Die Ergebnisse beider Analysatoren werden dann miteinander verglichen.

Ein Meßzyklus kann dabei einen oder mehrere Meßabschnitte umfassen, wobei die Genauigkeit der Messung mit steigender Zahl der durchgeführten Meßabschnitte steigt. Dazu wird vorteilhafter Weise in einem zweiten Meßabschnitt des Meßzyklus der berechnete Wert als Sollwert für einen einzustellenden Partialdruck des Gasgemisches aus Trägergas und einem dem Trägergas beizumischenden, dem zu messenden Gas gleichen Gas herangezogen und das Gasgemisch wird entsprechend dem Sollwert eingestellt und in einem Analysator wird der Istwert des Partialdruckes des Gasgemisches gemessen, wobei gleichzeitig dieses Gasgemisch dem Meßkreis zur Spülung und zur Aufnahme des zu messenden Gases zugeführt wird, dann wird der Partialdruck des Gasgemisches nach Gasaustausch mit der Metallschmelze gemessen und danach ein neuer Nährungswert berechnet. Durch diesen Meßabschnitt wird die Genauigkeit der Messung gesteigert. Durch Beimischen des dem zu messenden Gas gleichen Gases zu dem Trägergas weist dieses Trägergas von vornherein einen Gehalt des zu messenden Gases auf, der in der Nähe der Gleichgewichtskonzentration liegt, die bei Gasaustausch mit der Metallschmelze durch das Gas angestrebt wird.

Die Genauigkeit des Verfahrens kann dadurch weiter gesteigert werden, daß an den zweiten Meßabschnitt weitere Meßabschnitte anschließen, bei denen jeweils zumindest der neue Nährungsert als Sollwert für einen einzustellenden Partialdruck des Gasgemisches aus Trägergas und dem dem Trägergas beizumischenden, dem zu messenden Gas gleichen Gas herangezogen wird und dieses Gasgemisch entsprechend dem Sollwert eingestellt und in einem Analysator der Istwert des Partialdruckes dieses Gasgemisches gemessen wird, wobei gleichzeitig dieses Gasgemisch dem Meßkreis zur Spülung und zur Aufnahme des zu messenden Gases zugeführt wird, daß dann der Partialdruck des Gasgemisches nach Gasaustausch mit der Metallschmelze gemessen und daß danach ein weiterer neuer Näherungswert berechnet wird.

Für die Messung von Stickstoff in der Metallschmelze wird zweckmäßigerweise für einen Folge-Zyklus ein Sollwert für den Partialdruck im ersten Meßabschnitt dadurch berechnet, daß aus dem vorhergehenden Meßzyklus eine Konstante (B) herangezogen wird, die sich aus der Formel

$$(1) \qquad R_1 - S_1 = \frac{(X - S_1)}{B}$$

ergibt, wobei

$R_1 =$ Partialdruck des zu messenden Gases nach Gasaustausch in der Metallschmelze im ersten Meßabschnitt

$S_1 =$ Istwert des Partialdruckes des im ersten Meßabschnitt dem Analysator zusammen mit dem Trägergas direkt zugeführten dem zu messenden Gas gleichen Gases

$X =$ zu messender Wert des Partialdruckes des zu messenden Gases in der Metallschmelze

ist und daß sich die Bildung des Sollwertes ($M_2$) für das einzustellende Gasgemisch für den zweiten Meßabschnitt aus

$$(2) \qquad S_1 + B \times (R_1 - S_1) = M_2$$

ergibt.

Für die weitere Erhöhung der Genauigkeit dieser Messung ist es zweckmäßig, daß sich weitere Meßabschnitte anschließen, derart, daß sich ein Sollwert ($M_{j+1}$) für den Partialdruck im (j+1)ten Meßabschnitt aus der Formel

$$(3) \qquad M_{j+1} = \frac{S_j(R_j - S_j) - S_i(R_j - S_j)}{R_i - S_i - R_j - S_j} + S_j$$

ergibt, wobei i und j Maßzahlen für die Meßabschnitte darstellen, derart, daß der (j)te Meßabschnitt dem (i+n)ten Meßabschnitt entspricht, wobei i, j und n ganze Zahlen größer 0 sind. Das bedeutet, daß für die Berechnung von $M_{j+1}$ auf die Werte $S_j$ und $R_j$ aus dem unmittelbar vorhergehenden (j)ten Meßabschnitt zurückgegriffen wird sowie die Werte $S_i$ und $R_i$ aus beliebigen vorhergehenden Meßabschnitten benutzt werden.

Im Falle der Messung von Stickstoff in der Metallschmelze kann es vorteilhaft sein, daß aus der in einem Meßzyklus für den Folge-Meßzyklus berechneten Konstanten (B) für den jeweiligen Folge-Meßzyklus der Schwefelgehalt der Metallschmelze abgeleitet wird, wodurch gleichzeitig eine Aussage über eine weitere in der Schmelze enthaltene Komponente erhalten wird.

Für die Messung von Wasserstoff in der Metallschmelze ist es zweckmäßig, daß für einen Folge-Zyklus ein Sollwert für den Partialdruck im ersten Meßabschnitt dadurch berechnet wird, daß aus dem vorhergehenden Meßzyklus eine Konstante (B') herangezogen wird, die sich aus der Formel

$$(4) \qquad R_1 - S_1 = \frac{\sqrt{X} - \sqrt{S_1}}{B'}$$

ergibt, wobei

$R_1 =$ Partialdruck des zu messenden Gases nach Gasaustausch in der Metallschmelze im ersten Meßabschnitt

$S_1 =$ Istwert des Partialdruckes des im ersten Meßabschnitt dem Analysator zusammen mit dem Trägergas direkt zugeführten dem zu messenden Gas gleichen Gases

$X =$ zu messender Wert des Partialdruckes des zu messenden Gases in der Metallschmelze

ist und daß sich die Bildung des Sollwertes ($M_2$) für das einzustellende Gasgemisch für den zweiten Meßabschnitt aus

$$(5) \qquad [\sqrt{S_1} + B' (R_1 - S_1)]^2 = M_2$$

ergibt.

Für die weitere Erhöhung der Genauigkeit der Messung ist es im Fall der Messung von Wasserstoff in der Metallschmelze zweckmäßig, daß sich ein Sollwert ($M_{j+1}$) für den Partialdruck im (j+l)ten Meßabschnitt aus der Formel

$$(6) \qquad M_{j+1} = \frac{\sqrt{S_i}(R_i - S_i) - \sqrt{S_j}(R_i - S_i)}{R_j - S_j - R_i + S_i} + \sqrt{S_i}$$

ergibt, wobei i und j Meßzahlen für die Meßabschnitte darstellen, derart, daß der (j)te Meßabschnitt dem (i+n)ten Meßabschnitt entspricht, wobei i, j und n ganze Zahlen größer 0 sind. Dadurch kann ähnlich wie bei der Messung von Stickstoff durch die Aneinanderreihung mehrerer Meßabschnitte innerhalb eines Meßzyklus die Genauigkeit beliebig gesteigert werden.

Vorteilhaft ist es, daß aufgrund der Differenz zwischen dem Istwert des Partialdruckes des dem Analysator direkt zugeführten Gases und dem Sollwert für das einzustellende Gasgemisch eine Kalibrierung der Mischeinrichtung für die Zugabe des dem zu messenden Gas gleichen Gases zu dem Trägergas für den nächsten Meßabschnitt erfolgt. Dadurch wird die Zahl der notwendigen Meßabschnitte, die zur Erreichung einer hohen Meßgenauigkeit notwendig sind, vermindert.

Für den Fall, daß die Konzentration des zu messenden Gases sehr gering ist, genügt es, dem Trägergas auch eine sehr geringe Menge des dem zu messenden Gas gleichen Gases beizumischen. Um eine bessere Dosierbarkeit zu erzielen, ist es zweckmäßig, daß das dem Trägergas beigemischte Gas ein Gasgemisch aus einem dem Trägergas gleichen Gas und einem dem zu messenden Gas gleichen Gas ist.

Zur Verbesserung der Kalibrierung des Analysators ist es vorteilhaft, daß das Trägergas und/oder das Gemisch aus Trägergas und dem beizumischenden Gas vor der direkten Zuführung zu dem Analysator durch ein Filter von Fremdstoffen, wie beispielsweise von Wasser, gereinigt wird.

Zur schnelleren Beseitigung von Restgasen ist es zweckmäßig, daß mit dem Trägergas oder dem Gasgemisch aus Trägergas und dem beizumischenden Gas vor Einleitung in den Analysator und/oder den Meßkreis pulsierend gespült wird und dadurch die aus vorherigen Meßzyklen oder Meßabschnitten vorhandenen Restgase rasch und vollständig entfernt werden.

Nachstehend wir die Erfindung in einem Ausführungsbeispiel anhand einer Zeichnung näher erläutert. In der Zeichnung zeigen

Figur 1   eine schematische Darstellung der Gasführung und

Figur 2   eine im Analysator aufgezeichnete Meßkurve.

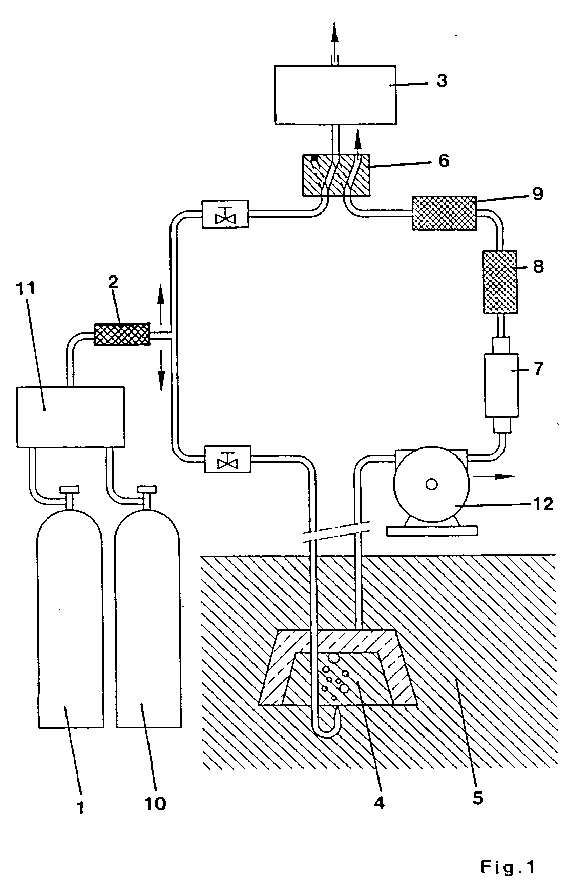

Die in Figur 1 schematisch dargestellte Gasführung wiederspiegelt den wesentlichen Meßaufbau, wie er für die Messung von Stickstoff in einer Metallschmelze, beispielsweise in einer Stahlschmelze, Verwendung findet. Dafür wird aus einer Trägergasquelle 1 Helium als Trägergas durch ein Filter 2, in welchem unter Umständen in dem Trägergas vorhandenes Wasser oder andere Fremdstoffe herausgefiltert werden, auf direktem Weg in den Analysator 3 zur Kalibrierung und gleichzeitig in den Meßkreis mit der Meßsonde 4 geleitet. Dadurch wird gleichzeitig mit der Kalibrierung des Analysators 3 der Meßkreis gespült und in der Meßsonde 4 das zu messende Gas aus der Metallschmelze 5 aufgenommen und an ein dem Analysator 3 vorgeschaltetes Mehrwegeventil 6 geführt. Zur Zeit des Kalibrierens des Analysators 3 verbindet das Mehrwegventil 6 die Trägergasquelle 1 direkt mit dem Analysator 3 während das den Meßkreis durchlaufende Gas an dem Mehrwegventil 6 nach außen abgeführt wird. Nach Kalibrierung des Analysators 3 schaltet das Mehrwegventil 6 um, so das das den Meßkreis durchlaufende Gasgemisch, daß das zu messende Gas enthält, dem Analysator 3 zur Auswertung zugeführt wird. Als Analysator 3 wird ein Katharometer verwendet. Dabei wird üblicherweise nur ein Teil des den Meßkreis durchlaufenden Gasgemisches dem Katharometer zugeführt.

Da mit dem Stickstoff aus der Metallschmelze 5 auch Wasserstoff und Kohlenmonoxid aufgenommen werden, wird das Gasgemisch nach dem Gasaustausch in der Metallschmelze 5 durch eine Oxidationssäule 7 geleitet, in der Wasserstoff zu Wasser und Kohlenmonoxid zu Kohlendioxid oxidiert werden. Wasser und Kohlendioxid werden in Filtern 8 und 9 herausgefiltert, so daß nur der aus der Metallschmelze aufgenommene Stickstoff in dem den Meßkreis durchlaufenden Gas verbleibt und dieses Gasgemisch in den Analysator 3 geführt wird.

Die Kontaktzeit des durch den Meßkreis geleiteten Gases mit der Schmelze ist sehr kurz, ebenso ist die Kontaktfläche zwischen den Gasblasen und der Schmelze gering, so daß nur eine begrenzte Gasmenge zwischen dem dem Meßkreis zugeleiteten Gas und der Schmelze ausgetauscht werden kann. Daher kann bei dem vorstehend beschriebenen Meßabschnitt nur ein erster Näherungswert für den Partialdruck des zu messenden Gases bestimmt werden.

Dieser erste Näherungswert ist ein Näherungswert für den Partialdruck des zu messenden Gases, wie er nach Erreichen des Gleichgewichtszustandes in der Metallschmelze 5 gemessen würde. Er ergibt sich aus der Gleichung (1), wobei X diesen Näherungswert darstellt, $R_1$ gibt den real gemessenen Partialdruck des zu messenden Gases nach Gasaustausch in der Metallschmelze 5 im ersten Meßabschnitt wieder, $S_1$ ist der Istwert des Partialdruckes des im ersten Meßabschnitt dem Analysator 3 direkt zugeführten Gases oder Gasgemischen, wobei theoretisch $S_1$ gleich 0 gilt, da im ersten Meßabschnitt das reine Trägergas direkt dem Analysator 3 zugeleitet wird. Die Konstante B ist eine Größe, die entweder willkürlich festgelegt werden kann, wobei je nach Abweichung der willkürlich festgelegten Konstante B von den realen Verhältnissen in der Metallschmelze 5 die Zahl der für eine genaue Messung erforderlich werdenden aufeinander folgenden Meßabschnitte ansteigen kann, wie im folgenden beschrieben wird. Die Konstante B kann auch aus einem vorhergehenden Meßzyklus gewonnen werden, unter der Voraussetzung, daß die Verhältnisse in der Metallschmelze 5 des vorhergehenden Meßzyklus und die Verhältnisse in der aktuell zu messenden Metallschmelze 5 nicht wesentlich voneinander abweichen.

Da der erste Näherungswert, wie beschrieben, in der Regel für eine genaue Messung nicht ausreicht, wird in einem zweiten Meßabschnitt dem Trägergas (Helium) aus einer weiteren Gasquelle 10, die ein dem zu messenden Gas gleiches Gas, also Stickstoff, oder ein Gemisch aus Helium und Stickstoff enthält, dieses in einem Gasmixer 11 beigemischt. Mit diesem Gasgemisch wird der erste Meßabschnitt wiederholt, es wird also erst dem Analysator 3 direkt und gleichzeitig dem Meßkreis dieses Gasgemisch zugeführt, nach Kalibrierung des Analysators 3 wird das Mehrwegventil 6 umgeschaltet, so daß das Gasgemisch nach Aufnahme des zu messenden Gases in der Metallschmelze dem Analysator 3 zugeleitet wird. Zur Unterstützung der Gasführung durch den Meßkreis wird eine Pumpe 12 verwendet. Das Filter 2 kann unmittelbar nach dem Gasmixer 11 oder beispielsweise auch zwischen Mehrwegventil 6 und Analysator 3 angeordnet sein. Im letztgenannten Fall kann das Filter 9 zur Filterung des Wassers im Meßkreis entfallen.

Der Anteil des dem Helium beizumischenden Stickstoffs aus der Gasquelle 10 soll möglichst nahe der Gleichgewichtskonzentration des Stickstoffs in der Metallschmelze sein. Dieser Sollwert $M_2$ für das einzustellende Gasgemisch bestimmt sich aus der Gleichung (2). Der Sollwert $M_2$ wird um so genauer den Gleichgewichtsbedingungen in der Metallschmelze entsprechen, je genauer die Konstante B den realen Verhältnissen entsprechend gewählt wurde. Die Genauigkeit des gemessenen Partialdruckes des zu messenden Gases nach Gasaustausch in der Metallschmelze ergibt sich aus einem Vergleich dieses Partialdruckes mit dem Sollwert. Liegt die erforderliche Genauigkeit außerhalb der vorgegebenen Toleranzen, so schließt sich ein weiterer Meßabschnitt an, bei dem sich der Sollwert $M_3$ aus Gleichung (3) ergibt. Wenn die Differenz zwischen Sollwert und Partialdruck des zu messenden Gases nach Gasaustausch in der Metallschmelze innerhalb der vorgegebenen Toleranzen liegt, so bedeutet dies, daß dem Trägergas (Helium) eine solche Menge des dem zu messenden Gas gleichen Gases (Stickstoff) in dem Gasmixer 11 beigemischt wurde, daß der Stickstoff-Partialdruck dieses Gasgemisches dem Stickstoff-Partialdruck in der Metallschmelze entspricht, so daß in der Metallschmelze kein Gasaustausch mit dem in die Metallschmelze eingeleiteten Gasgemisch mehr stattfindet. Dieses Verfahren ist also ein Näherungsverfahren, bei dem versucht wird, ein Gasgemisch vorzugeben, dessen Stickstoffanteil dem Stickstoffanteil in der Metallschmelze entspricht, so daß ein langwieriger Gasaustausch bis zum Erreichen des Gleichgewichtszustandes überflüssig wird und lediglich festgestellt werden muß, ob überhaupt noch ein Gasaustausch stattfindet.

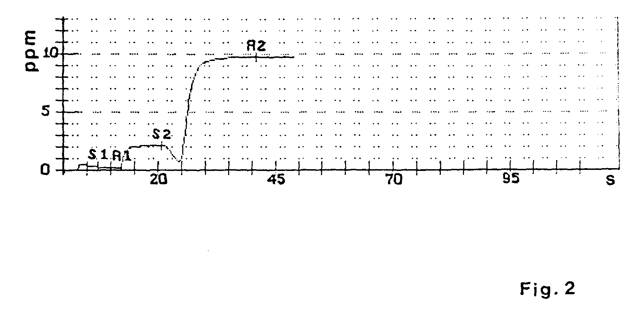

In Figur 2 ist eine Meßkurve dargestellt, wie sie bei dem oben beschriebenen Verfahren aufgezeichnet wurde. Im ersten Meßabschnitt wird dem Helium kein Stickstoff beigemischt. $S_1$ weicht trotzdem etwas von 0 ab, da sich in der Regel Restgase in den Gasleitungen befinden. Diese Restgase werden allerdings sehr schnell durch pulsierende Spülung der Gasleitungen abgesaugt. Die am Punkt $R_1$ beginnende Messung des Partialdruck $R_1$ des zu messenden Gases nach Gasaustausch in der Metallschmelze ist sehr niedrig, da der Gasaustausch sehr schnell vonstatten geht und aufgrund des Fehlens von Stickstoff in dem Trägergas die Gleichgewichtskonzentration bei weitem nicht erreicht wird. Entsprechend dem errechneten Sollwert $M_2$ wird deshalb im Punkt $S_2$ ein weiterer Meßabschnitt gestartet, bei dem eine dem Sollwert $M_2$ entsprechende Stickstoffmenge dem Helium im Gasmixer 11 beigemischt wird. Dieses Gasgemisch wird dann dem Analysator 3 direkt zugeführt, die Kurve steigt bei der Messung dieses Gasgemisches steil an bis ein konstanter Meßwert in dem Analysator 3 erreicht ist. In diesem, in Figur 2 mit $R_2$ bezeichneten Punkt schaltet das Mehrwegventil 6 um, so daß das Gasgemisch aus dem Meßkreis dem Analysator 3 zugeleitet und gemessen wird. Die Differenz zwischen dem Sollwert $S_2$ und dem Partialdruck $R_1$ des zu messenden Gases nach Gasaustausch in der Metallschmelze ist vernachlässigbar, so daß die Messung abgebrochen wird.

Eine weitere Verkürzung der Meßzeit ist dadurch möglich, daß der Wert $S_2$ durch Extrapolation ermittelt wird und dadurch die Umschaltung des Mehrwegventils 6 eher erfolgen kann. Der Wert $R_2$ kann ebenfalls durch Extrapolation bestimmt werden, so daß die Zeit für die Ermittlung des Stickstoffgehaltes in der Metallschmelze 5 insgesamt verkürzt werden kann.

In ähnlicher Weise ist die Bestimmung von Wasserstoff in Metallschmelzen, beispielsweise in Aluminiumschmelze möglich, wobei in diesem Fall die Gleichungen (4), (5) und (6) verwendet werden. Bei der Bestimmung von Wasserstoff wird als Trägergas bevorzugt Argon oder Stickstoff verwendet. Weiterhin ist es bei der Bestimmung von Wasserstoff nicht unbedingt erforderlich, weitere Gasbestandteile durch Filterung aus dem aus der Metallschmelze kommenden Gasgemisch zu entfernen.

**Patentansprüche**

1. Verfahren zur Bestimmung der Konzentration eines Gases in einer Metallschmelze mittels eines Trägergases, das mindestens einem Analysator (3) wahlweise entweder zur Kalibrierung auf direktem Weg oder zur Messung über einen Meßkreis mit einer in die Metallschmelze eingetauchten Meßsonde (4) zugeführt werden kann, wobei das Trägergas zur Messung mit der Metallschmelze in einen Gasaustausch tritt und dieses Gasgemisch dem Analysator (3) zugeführt wird, dadurch gekennzeichnet, daß für einen Meßzyklus in einem Meßabschnitt das Trägergas gleichzeitig sowohl direkt dem Analysator (3) zur Kalibrierung als auch dem Meßkreis zur Spülung und zur Aufnahme des zu messenden Gases zugeführt wird derart, daß das zu messende Gas am Analysator (3) bereitsteht, daß anschließend das Trägergas über den Meßkreis mindestens teilweise dem mindestens einen Analysator (3) zugeführt wird und daß ein Partialdruck als erster Näherungswert für den Partialdruck des zu messenden Gases in der Metallschmelze (5) nach dem Gasaustausch mit der Metallschmelze (5) berechnet wird.

2. Verfahren nach Anspruch 1, dadurch gekennzeichnet, daß in einem zweiten Meßabschnitt des Meßzyklus der berechnete Wert als Sollwert für einen einzustellenden Partialdruck des Gasgemisches aus Trägergas und einem dem Trägergas beizumischenden, dem zu messenden Gas gleichen Gas herangezogen wird und das Gasgemisch entsprechend dem Sollwert eingestellt und in einem Analysator (3) der Istwert des Partialdruckes dieses Gasgemisches gemessen wird, wobei gleichzeitig dieses Gasgemisch dem Meßkreis zur Spülung und zur Aufnahme des zu messenden Gases zugeführt wird, daß dann der Partialdruck des Gasgemisches nach Gasaustausch mit der Metallschmelze (5) gemessen wird und daß danach ein neuer Näherungswert berechnet wird.

3. Verfahren nach Anspruch 2, dadurch gekennzeichnet, daß an den zweiten Meßabschnitt weitere Meßabschnitte anschließen, bei denen jeweils zumindest der neue Näherungswert als Sollwert für einen einzustellenden Partialdruck des Gasgemisches aus Trägergas und dem dem Trägergas beizumischenden, dem zu messenden Gas gleichen Gas herangezogen wird und dieses Gasgemisch entsprechend dem Sollwert eingestellt und in einem Analysator (3) der Istwert des Partialdruckes dieses Gasgemisches gemessen wird, wobei gleichzeitig dieses Gasgemisch dem Meßkreis zur Spülung und zur Aufnahme des zu messenden Gases zugeführt wird, daß dann der Partialdruck des Gasgemisches nach Gasaustausch mit der Metallschmelze (5) gemessen wird und daß danach ein weiterer neuer Näherungswert berechnet wird.

4. Verfahren nach einem der Ansprüche 1 bis 3, dadurch gekennzeichnet, daß für die Messung von Stickstoff in der Metallschmelze (5) für einen Folge-Meßzyklus ein Sollwert für den Partialdruck im ersten Meßabschnitt dadurch berechnet wird, daß aus dem vorhergehenden Meßzyklus eine Konstante (B) herangezogen wird, die sich aus der Formel

$$R_1 - S_1 = \frac{(X - S_1)}{B}$$

ergibt, wobei

$R_1$ = Partialdruck des zu messenden Gases nach Gasaustausch in der Schmelze im ersten Meßabschnitt

$S_1$ = Istwert des Partialdruckes des im ersten Meßabschnitt dem Analysator (3) zusammen mit dem Trägergas direkt zugeführten dem zu messenden Gas gleichen Gases

$X$ = zu messender Wert des Partialdruckes des zu messenden Gases in der Metallschmelze

ist und
daß sich die Bildung des Sollwertes ($M_2$) für das einzustellende Gasgemisch für den zweiten Meßabschnitt aus

$$S_1 + B \times (R_1 - S_1) = M_2$$

ergibt.

5. Verfahren nach Anspruch 4, dadurch gekennzeichnet, daß sich ein Sollwert ($M_{j+1}$) für den Partialdruck im (j+l)ten Meßabschnitt aus der Formel

$$M_{j+1} = \frac{S_j(R_j - S_j) - S_i (R_j - S_j)}{R_i - S_i - R_j - S_j} + S_j$$

ergibt, wobei i und j Maßzahlen für die Meßabschnitte darstellen, derart, daß der (j)te Meßabschnitt dem (i+n)ten Meßabschnitt entspricht, wobei i, j und n ganze Zahlen größer Null sind.

6. Verfahren nach Anspruch 4 oder 5, dadurch gekennzeichnet, daß aus der in einem Meßzyklus für den Folge-Meßzyklus berechneten Konstanten (B) für den jeweiligen Folge-Meßzyklus der Schwefelgehalt der Metallschmelze (5) abgeleitet wird.

7. Verfahren nach einem der Ansprüche 1 bis 3, dadurch gekennzeichnet, daß für die Messung von Wasserstoff in der Metallschmelze (5) für einen Folge-Zyklus ein Sollwert für den Partialdruck im ersten Meßabschnitt dadurch berechnet wird, daß aus dem vorhergehenden Meßzyklus eine konstante (B') herangezogen wird, die sich aus der Formel

$$R_1 - S_1 = \frac{\sqrt{X} - \sqrt{S_1}}{B'}$$

ergibt, wobei

$R_1$ = Partialdruck des zu messenden Gases nach Gasaustausch in der Schmelze im ersten Meßabschnitt

$S_1$ = Istwert des Partialdruckes des im ersten Meßabschnitt dem Analysator (3) zusammen mit dem Trägergas direkt zugeführten dem zu messenden Gas gleichen Gases

$X$ = zu messender Wert des Partialdruckes des zu messenden Gases in der Metallschmelze

ist und
daß sich die Bildung des Sollwertes $M_2$ für das einzustellende Gasgemisch für den zweiten Meßabschnitt aus

$$[ \sqrt{S_1} + B' (R_1 - S_1) ]^2 = M_2$$

ergibt.

8. Verfahren nach Anspruch 7, dadurch gekennzeichnet, daß sich ein Sollwert ($M_{j+1}$) für den Partialdruck im (j+1)ten Meßabschnitt aus der Formel

$$M_{j+1} = \frac{\sqrt{S_i} (R_i - S_i) - \sqrt{S_j} (R_i - S_i)}{R_j - S_j - R_i + S_i} + \sqrt{S_i}$$

ergibt, wobei i und j Maßzahlen für die Meßabschnitt darstellen, derart, daß der (j)te Meßabschnitt dem (i+n)ten Meßabschnitt entspricht, wobei i, j und n ganze Zahlen größer 0 sind.

9. Verfahren nach einem der Ansprüche 1 bis 8, dadurch gekennzeichnet, daß aufgrund der Differenz zwischen dem Istwert des Partialdruckes des dem Analysator (3) direkt zugeführten Gases und dem Sollwert für das einzustellende Gasgemisch eine Kalibrierung der Mischeinrichtung (11) für die Zugabe des dem zu messenden Gas gleichen Gases zu dem Trägergas für den nächsten Meßabschnitt erfolgt.

10. Verfahren nach einem der Ansprüche 2 bis 9, dadurch gekennzeichnet, daß das dem Trägergas beigemischte Gas ein Gasgemisch aus einem dem Trägergas gleichen Gas und einem dem zu messenden Gas gleichen Gas ist.

11. Verfahren nach einem der Ansprüche 1 bis 10, dadurch gekennzeichnet, daß das Trägergas und/oder das Gemisch aus Trägergas und dem beizumischenden Gas vor der direkten Zuführung zu dem Analysator (3) durch ein Filter (2) von Fremdstoffen gereinigt wird.

12. Verfahren nach einem der Ansprüche 1 bis 11, dadurch gekennzeichnet, daß mit dem Trägergas oder dem Gasgemisch aus Trägergas und beizumischendem Gas vor Einleitung in den Analysator (3) und/oder den Meßkreis pulsierend gespült wird.

**Claims**

1. A method for determining the concentration of a gas in a metal melt by means of a carrier gas, which can be supplied to at least one analyser (3) optionally either for calibration directly or for measurement via a measurement circuit with a measurement probe (4) which is immersed into the metal melt, in which the carrier gas enters into a gas exchange for measurement with the metal melt and this gas mixture is supplied to the analyser (3), characterised in that for a measurement cycle in one measurement section the carrier gas is supplied simultaneously both directly to the analyser (3) for calibration and also to the measurement circuit for flushing and for receiving the gas which is to be measured, such that the gas which is to be measured is available at the analyser (3), that the carrier gas is then supplied via the measurement circuit at least partially to the at least one analyser (3) and that a partial pressure is calculated as first approximate value for the partial pressure of the gas which is to be measured in the metal melt (5) after the gas exchange with the metal melt (5).

2. A method according to Claim 1, characterised in that in a second measurement section of the measurement cycle, the calculated value is drawn upon as nominal value for a partial pressure, which is to be adjusted, of the gas mixture of carrier gas and of a gas which is to be admixed to the carrier gas and which is identical to the gas which is to be measured, and the gas mixture is adjusted according to the nominal value and in an analyser (3) the actual value of the partial pressure of this gas mixture is measured, in which at the same time this gas mixture is supplied to the measurement circuit for flushing and for receiving the gas which is to be measured, that then the partial pressure of the gas mixture is measured after gas exchange with the metal melt (5) and that thereafter a new approximate value is calculated.

3. A method according to Claim 2, characterised in that joining on to the second measurement section are further measurement sections, in which in each case at least the new approximate value is drawn upon as nominal value for a partial pressure, which is to be adjusted, of the gas mixture of carrier gas and of the gas which is to be admixed to the carrier gas and which is identical to the gas which is to be measured, and this gas mixture is adjusted according to the nominal value and in an analyser (3) the actual value of the partial pressure of this gas mixture is measured, in which at the same time this gas mixture is supplied to the measurement circuit for flushing and for receiving the gas which is to be measured, that then the partial pressure of the gas mixture is measured after gas exchange with the metal melt (5) and that thereafter a further new approximate value is calculated.

4. A method according to one of Claims 1 to 3, characterised in that for the measurement of nitrogen in the metal melt (5) for a successive measurement cycle a nominal value is calculated for the partial pressure in the first measurement section, that from the preceding measurement cycle is obtained a constant (B), which results from the formula

$$R_1 - S_1 = \frac{(X - S_1)}{B}$$

in which

$R_1 =$ partial pressure of the gas to be measured after gas exchange in the melt in the first measurement section

$S_1 =$ actual value of the partial pressure of the gas which is supplied directly in the first measurement section to the analyser (3) together with the carrier gas and which is identical to the gas which is to be measured

$X =$ value, to be measured, of the partial pressure of the gas to be measured in the metal melt

and

that the formation of the nominal value ($M_2$) for the gas mixture, to be adjusted, for the second measurement section results from

$$S_1 + B \times (R_1 - S_1) = M_2.$$

5. A method according to Claim 4, characterised in that a nominal value ($M_{j+1}$) for the partial pressure in the (j+1)th measurement section results from the formula

$$M_{j+i} \quad \frac{S_j(R_j - S_j) - S_i (R_j - S_j)}{R_i - S_i - R_j - S_j} + S_j$$

in which i and j represent coefficients of measure for the measurement sections, such that the (j)th measurement section corresponds to the (i+n)th measurement section, in which i, j and n are whole numbers greater than zero.

6. A method according to Claim 4 or 5, characterised in that the sulphur content of the metal melt (5) is derived from the constant (B), calculated in a measurement cycle for the successive measurement cycle, for the respective successive measurement cycle.

7. A method according to one of Claims 1 to 3, characterised in that for the measurement of hydrogen in the metal melt (5) for a successive cycle a nominal value for the partial pressure in the first measurement section is calculated, that from the preceding measurement cycle is obtained a constant (B'), which results from the formula

$$R_1 - S_1 = \frac{\sqrt{X} - \sqrt{S_1}}{B'}$$

in which

$R_1 =$ partial pressure of the gas to be measured after gas exchange in the melt in the first measurement section

$S_1 =$ actual value of the partial pressure of the gas supplied directly in the first measurement section to the analyser (3) together with the carrier gas and identical to the gas which is to be measured

$X =$ value, to be measured, of the partial pressure of the gas to be measured in the metal melt

and
that the formation of the nominal value $M_2$ for the gas mixture, to be adjusted, for the second measurement section, results from

$$[\sqrt{S_1} + B' (R_1 - S_1)]^2 = M_2.$$

8. A method according to Claim 7, characterised in that a nominal value ($M_{j+i'}$) for the partial pressure in the (j+1)th measurement section results from the formula

$$M_{j+i} = \frac{\sqrt{S_i} (R_i - S_i) - \sqrt{S_j} (R_i - S_i)}{R_j - S_j - R_i - S_i} + \sqrt{S_i}$$

in which i and j represent coefficients of measure for the measurement section, such that the (j)th measurement section corresponds to the (i+n)th measurement section, in which i, j and n are whole numbers greater than 0.

9. A method according to one of Claims 1 to 8, characterised in that on the basis of the difference between the actual value of the partial pressure of the gas supplied directly to the analyser (3) and the nominal value for the gas mixture which is to be adjusted, a calibration takes place of the mixing arrangement (11) for the addition of the gas, identical to the gas to be measured, to the carrier gas for the next measurement section.

10. A method according to one of Claims 2 to 9, characterised in that the gas which is admixed to the carrier gas is a gas mixture of a gas identical to the carrier gas and of a gas identical to the gas which is to be measured.

11. A method according to one of Claims 1 to 10, characterised in that the carrier gas and/or the mixture of carrier gas and the gas which is to be admixed is cleaned of foreign matter by a filter (2) before being supplied directly to the analyser (3).

12. A method according to one of Claims 1 to 11, characterised in that an intermittent flushing is carried out with the carrier gas or the gas mixture of carrier gas and gas to be admixed before introduction into the analyser (3) and/or into the measurement circuit.

**Revendications**

1. Procédé de détermination de la concentration d'un gaz dans une masse de métal en fusion au moyen d'un gaz vecteur qui peut être envoyé à au moins un analyseur (3) au choix pour l'étalonnage par voie directe ou pour la mesure par l'intermédiaire d'un circuit de mesure avec une sonde de mesure (4) plongée dans la masse de métal en fusion, le gaz vecteur étant soumis pour la mesure à un échange de gaz avec la masse de métal en fusion et ce mélange gazeux étant envoyé à l'analyseur (3), caractérisé en ce que, pour un cycle de mesure, dans une phase de mesure, le gaz de vecteur est envoyé simultané directement à l'analyseur (3) pour l'étalonnage et aussi au circuit de mesure pour le nettoyage et pour l'absorption du gaz qui doit être mesuré de telle manière que le gaz qui doit être mesuré est disponible au niveau de l'analyseur (3), qu'ensuite le gaz vecteur est envoyé par l'intermédiaire du circuit de mesure au moins en partie à au moins un analyseur (3) et qu'une pression partielle est calculée sous forme de première valeur approchée pour la pression partielle du gaz qui doit être mesuré dans la masse de métal en fusion (5) après l'échange de gaz avec la masse de métal en fusion (5).

2. Procédé selon la revendication 1, caractérisé en ce que, dans une seconde phase de mesure du cycle de mesure, la valeur calculée est choisie comme valeur de consigne pour une pression partielle qui doit être établie pour le mélange gazeux constitué par le gaz vecteur et un gaz identique au gaz qui doit être mesuré qui est mélangé au gaz vecteur, et le mélange gazeux est réglé en fonction de la valeur de consigne et la valeur effective de la pression partielle de ce mélange gazeux est mesurée dans un analyseur (3), ce mélange gazeux étant envoyé simultanément au circuit de mesure pour le nettoyage et pour l'absorption du gaz qui doit être mesuré, après quoi la pression partielle du mélange gazeux est mesurée après échange de gaz avec la masse de métal en fusion (5) puis une nouvelle valeur approchée est calculée.

3. Procédé selon la revendication 2, caractérisé en ce que la seconde phase de mesure est suivie par d'autres phases de mesure dans lesquelles, à chaque fois, au moins la nouvelle valeur approchée est choisie comme valeur de consigne pour une pression partielle qui doit être établie pour le mélange gazeux constitué par le gaz vecteur et le gaz identique au gaz qui doit être mesuré qui est mélangé au gaz vecteur, et ce mélange gazeux est réglé en fonction de la valeur de consigne, et la valeur effective de la pression partielle de ce mélange gazeux est mesurée dans un analyseur (3), ce mélange gazeux étant envoyé simultanément dans le circuit de mesure pour le nettoyage et pour l'absorption du gaz qui doit être mesuré, puis la pression partielle du mélange gazeux est mesurée après échange de gaz avec la masse de métal en fusion (5) et ensuite une nouvelle valeur approchée supplémentaire est calculée.

4. Procédé selon l'une des revendications 1 à 3, caractérisé en ce que, pour la mesure de l'azote dans la masse de métal en fusion (5), pour un cycle de mesure séquentiel, une valeur de consigne pour la pression partielle dans la première phase de mesure est calculée par le fait qu'une constante (B) est choisie à partir du cycle de mesure précédent, qui est donnée par la formule :

$$R_1 - S_1 = \frac{(X - S_1)}{B}$$

dans laquelle

$R_1$ = pression partielle du gaz qui doit être mesuré après échange de gaz dans la masse en fusion dans la première phase de mesure

$S_1$ = valeur effective de la pression partielle du gaz identique au gaz qui doit être mesuré qui est envoyé directement à l'analyseur (3) en même temps que le gaz vecteur dans la première phase de mesure

$X$ = valeur qui doit être mesurée de la pression partielle du gaz qui doit être mesuré dans la masse de métal en fusion

et en ce que la formation de la valeur de consigne $(M_2)$ pour le mélange gazeux qui doit être réglé pour la seconde phase de mesure est donnée par

$$S_1 + B \times (R_1 - S_1) = M_2$$

5. Procédé selon la revendication 4, caractérisé en ce qu'une valeur de consigne $(M_{j+1})$ pour la pression partielle dans la (j+1)ième phase de mesure est donnée par la formule

$$M_{j+1} = \frac{S_j(R_j - S_j) - S_i(R_j - S_j)}{R_i - S_i - R_j + S_j} + S_j$$

dans laquelle i et j représentent des nombres de mesure pour les phases de mesure, de telle manière que la jième phase de mesure correspond à la (i+n)ième phase de mesure, i, j et n étant des nombres entiers supérieurs à 0.

6. Procédé selon la revendication 4 ou 5, caractérisé en ce que des constantes (B) calculées dans un cycle de mesure pour le cycle de mesure successif est déduite la teneur en soufre de la masse de métal en fusion (5) pour le cycle de mesure successif correspondant.

7. Procédé selon l'une des revendications 1 à 3, caractérisé en ce que, pour la mesure de l'hydrogène dans la masse de métal en fusion (5) pour un cycle successif, une valeur de consigne pour la pression partielle est calculée dans la première phase de mesure de telle manière qu'à partir du cycle de mesure précédent est choisie une constante (B') donnée par la formule

$$R_1 - S_1 = \frac{\sqrt{X} - \sqrt{S_1}}{B'}$$

dans laquelle

$R_1$ = pression partielle du gaz qui doit être mesuré après échange de gaz dans la masse en fusion dans la première phase de mesure

$S_1$ = valeur effective de la pression partielle du gaz identique au gaz qui doit être mesurée qui est envoyé directement dans la première phase de mesure à l'analyseur (3) en même temps que le gaz vecteur

$X$ = valeur qui doit être mesurée de la pression partielle du gaz qui doit être mesuré dans la masse de métal

et que la formation de la valeur de consigne ($M_2$) pour le mélange gazeux qui doit être réglé pour la seconde phase de mesure est donnée par

$$[ \sqrt{S_1} + B' (R_1 - S_1)]^2 = M_2$$

8. Procédé selon la revendication 7, caractérisé en ce qu'une valeur de consigne ($M_{j+1}$) pour la pression partielle dans la (j+1)ième phase de mesure est donnée par la formule

$$M_{j+1} = \frac{\sqrt{S_i} (R_i - S_i) - \sqrt{S_j} (R_i - S_i)}{R_j - S_j - R_i + S_i} + \sqrt{S_i}$$

dans laquelle i et j représentent des nombres de mesure pour la phase de mesure de manière que la jième phase de mesure corresponde à la (i+n)ième phase de mesure, i, j et n étant des nombres entiers supérieurs à 0.

9. Procédé selon l'une des revendications 1 à 8, caractérisé en ce qu'un étalonnage du dispositif de mélange (11) pour l'addition au gaz vecteur du gaz identique au gaz qui doit être mesuré est réalisé pour la phase de mesure suivante sur la base de la différence entre la valeur effective de la pression partielle du gaz envoyé directement à l'analyseur (3) et la valeur de consigne pour le mélange gazeux qui doit être réglé.

10. Procédé selon l'une des revendications 2 à 9, caractérisé en ce que le gaz mélangé au gaz vecteur est un mélange gazeux constitué par un gaz identique au gaz vecteur et par un gaz identique au gaz qui doit être mesuré.

11. Procédé selon l'une des revendications 1 à 10, caractérisé en ce que le gaz vecteur et/ou le mélange de gaz vecteur et de gaz qui doit être mélangé est purifié des substances étrangères par un filtre (2) avant l'envoi direct à l'analyseur (3).

12. Procédé selon l'une des revendications 1 à 11, caractérisé en ce qu'un nettoyage par impulsions est réalisé avec le gaz vecteur ou le mélange gazeux constitué par le gaz vecteur et le gaz qui doit être mélangé avant l'introduction dans l'analyseur (3) et/ou le circuit de mesure.

Fig.1

Fig. 2